# EUROPÄISCHE PATENTSCHRIFT

(11) **EP 1 236 464 B1**
(45) Veröffentlichungstag und Bekanntmachung des Hinweises auf die Patenterteilung: **23.01.2008**
(21) Anmeldenummer: 02004066.3
(22) Anmeldetag: 23.02.2002
(51) Int. Cl.: A61K 8/81, A61Q 19/00, A61K 47/32

(54) **Tensidefreie kosmetische, dermatologische und pharmazeutische Mittel**
Cosmetic, dermatological or pharmaceutical composition without surfactants
Composition cosmétique, dermatologique et pharmaceutique sans tensio-actifs

(30) Priorität: 03.03.2001 DE 10110336
(43) Veröffentlichungstag der Anmeldung: 04.09.2002
(73) Patentinhaber: Clariant Produkte (Deutschland) GmbH, 65929 Frankfurt am Main (DE)
(72) Erfinder: Löffler, Matthias, Dr., 65527 Niedernhausen (DE); Morschhäuser, Roman, Dr., 55122 Mainz (DE); Hornung, Michael, 60326 Frankfurt (DE)

(56) Entgegenhaltungen:
- EP-A- 0 750 899
- EP-A- 0 815 844
- EP-A- 1 028 129
- WO-A-02/43686
- WO-A-02/43687
- PATENT ABSTRACTS OF JAPAN vol. 2000, no. 21, 3. August 2001 (2001-08-03) & JP 2001 115135 A (SHISEIDO CO LTD), 24. April 2001 (2001-04-24)

## Beschreibung

Die vorliegende Erfindung betrifft tensidfreie kosmetische, pharmazeutische und dermatologische Mittel, enthaltend vernetzte wasserlösliche oder wasserquellbare Copolymerisate auf Basis von Acrylamidoalkylsulfonsäuren und cyclischen N-Vinylcarbonsäureamiden oder cyclischen und linearen N-Vinylcarbonsäureamiden.

Kosmetische Hautpflegemittel haben in erster Linie die Aufgabe die natürliche Funktion der Haut als Barriere gegen Umwelteinflüsse (z.B. Schmutz, Chemikalien, Mikroorganismen) und gegen den Verlust von körpereigenen Stoffen (z.B. Wasser, natürliche Fette, Elektrolyte) zu stärken bzw. wiederherzustellen.
Ziel der Hautpflege ist es ferner, den durch tägliches Waschen verursachten Fett-und Wasserverlust der Haut auszugleichen.
Medizinische Formulierungen für die Haut enthalten in der Regel ein oder mehrere Medikamente in wirksamer Konzentration.
Die gegenwärtig verwendeten kosmetischen oder dermatologischen Zusammensetzungen liegen meistens in Form von Öl-in-Wasser-Emulsionen oder Wasser-in-Öl-Emulsionen vor.
Wasser-in-Öl-Emulsionen enthalten eine kontinuierliche Öl-Phase und lassen zu, dass sich an der Hautoberfläche ein Fettfilm bildet, der den transepidermalen Wasserverlust vermeidet und die Haut vor externen Aggressionen schützt.
Solche Emulsionen eignen sich besonders zum Schutz der Haut und zur Behandlung trockener Haut.
Öl-in-Wasser-Emulsionen dagegen verleihen der Haut beim Auftragen ein weiches, weniger fettiges und angenehmes Gefühl.

Die Emulsionen werden im allgemeinen durch Einarbeitung emulgierender Tenside vom Öl-in-Wasser-Typ (O/W) oder vom Wasser-in-ÖI-Typ (W/O) stabilisiert.
Um eine hinreichende Stabilität der Emulsionen zu erreichen, müssen derartige Tenside jedoch meist in Mengen bis zu 10 Gew.-%, bezogen auf das

Gesamtgewicht der Emulsionen, zuzugeben werden. Emulsionen ohne Tenside zeigen im allgemeinen eine unzureichende Stabilisierung der Ölkomponenten, was zur Koagulation und Separation der Ölphasen führt.

Nachteilig am Einsatz der Tenside ist, dass sie zu einer Reizung der Haut, den Augen und der Kopfhaut führen können oder im Einzelfall sogar eine allergische Reaktion auslösen können. So ist z.B. bekannt, dass bestimmte Tenside bei gleichzeitiger Exposition gegenüber Sonnenlicht Lichtdermatosen auslösen können. Ein weiterer Nachteil ist, dass hohe Tensidkonzentrationen zu einem rauen, klebrigen oder zähen Gefühl der Mittel führen können oder die Mittel kompakt und schwer erscheinen lassen. Darüber hinaus müssen die Tenside in Abhängigkeit von der Polarität der Öle ausgewählt werden, so dass die Vielfalt der Formulierungen beschränkt ist.

Die Anwender von Emulsionen sind daher ständig bemüht den Tensidgehalt zu reduzieren, um die Verträglichkeit der Emulsionen zu verbessern und ihre kosmetischen Eigenschaften zu optimieren.

Im Laufe der letzten Jahre etablierten sich Polymere auf dem Markt, die die Formulierung von tensidarmen oder sogar tensidfreien Emulsionen ermöglichen (WO 96/37180 und US 5736125). Bei diesen Polymeren handelt es sich um hydrophobe Modifikationen konventioneller Poly(meth)acrylate, die sowohl verdickende als auch emuigierende/dispergierende Eigenschaften aufweisen. Beispiele für kommerzielle Produkte sind ^{®}Pemulen TR-1 und TR-2 von BF-Goodrich und ^{®}Aculyn 22 und ^{®}Aculyn 28 von Rohm und Haas.
Da derart hydrophob modifizierte Polymere auf der Basis von (Meth)acrylsäure aufgebaut sind, besitzen sie folglich die Nachteile der Poly(meth)acrylate. Ein wesentlicher Nachteil von Verdickern auf Basis von Poly(meth)acrylsäure ist die starke pH-Abhängigkeit der Verdickungsleistung. So wird im allgemeinen eine hinreichende Viskosität nur dann aufgebaut, wenn der pH-Wert der Formulierung oberhalb von pH 6.0 eingestellt ist und somit die Poly(meth)acrylsäure in neutralisierter Form vorliegt.

In DE 44 25 268 werden emulgatorfreie, feindisperse Öl-in-Wasser Zubereitungen beschrieben, die Acrylsäurepolymere als Verdicker enthalten, die jedoch ebenfalls für saure Formulierungen ungeeignet sind.

In EP 815 844 werden kosmetische und/oder dermatologische Zusammensetzungen in Form einer Öl-in-Wasser-Emulsion beschrieben, die dadurch gekennzeichnet sind, dass sie mindestens ein vernetztes und zu mindestens 90 % neutralisiertes Poly(2-acrylamido-2-methylpropan-sulfonsäure)-Polymer und keinen grenzflächenaktiven Stoff enthalten.

Überraschend wurde nun gefunden, dass vernetzte Copolymere auf Basis von Acrylamidoalkylsulfonsäure und cyclischen N-Vinylcarbonsäureamiden oder cyclischen und linearen N-Vinylcarbonsäureamiden hervorragend als Verdicker, Stabilisator, Dispergator und Gleitmittel in tensidfreien kosmetischen, dermatologischen und pharmazeutischen Mitteln geeignet sind.
Vorteilhafterweise zeigen die Copolymere über einen weiten pH-Bereich, d.h. auch bei sauren pH-Werten, sehr gute Verdickungseigenschaften.

Gegenstand der Erfindung sind daher tensidfreie kosmetische, dermatologische und pharmazeutische Mittel, enthaltend mindestens ein Copolymer, bestehend im wesentlichen aus
a1) 1 bis 50 Gew.-% der wiederkehrenden Struktureinheit der Formel (1) 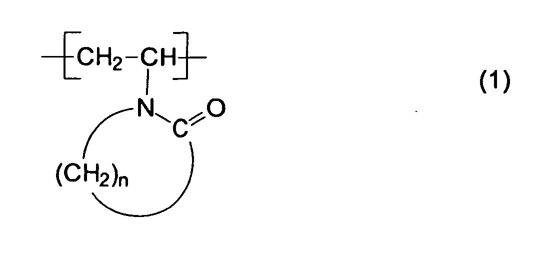 wobei n eine ganze Zahl von 2 bis 9 bedeutet
oder
a2) 1 bis 50 Gew.-% einer Mischung der wiederkehrenden Struktureinheit der Formel (1) und der wiederkehrenden Struktureinheit der Formel (2) 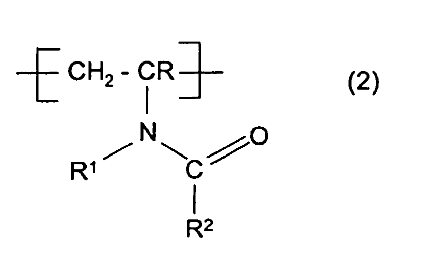 wobei R, R¹ und R² gleich oder verschieden sein können und Wasserstoff oder eine lineare oder verzweigte Alkyl- oder Alkenylgruppe mit jeweils 1 bis 30, bevorzugt 1 bis 20, insbesondere 1 bis 12 C-Atomen, bedeuten
und
b) 49,99 bis 98,99 Gew.-% der wiederkehrenden Struktureinheit der Formel (3) 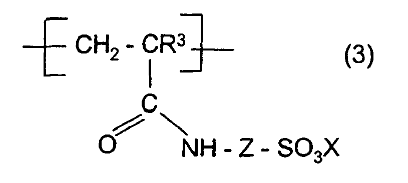 worin R³ Wasserstoff, Methyl oder Ethyl, Z (C₁-C₈)-Alkylen, n eine ganze Zahl von 2 bis 9 und X ein Ammonium-, Alkali- oder Erdalkali-lon bedeuten
und
c) 0,01 bis 8 Gew.-% vernetzenden Strukturen, die aus Monomeren mit mindestens zwei olefinischen Doppelbindungen hervorgegangen sind.

Das Mischungsverhältnis betreffend Struktureinheit a2) kann innerhalb beliebiger Grenzen variieren.

Bevorzugte Copolymere enthalten
2 bis 30 Gew.-%, besonders bevorzugt 3 bis 15 Gew.-%, der Struktureinheiten a1) oder a2), bevorzugt der Struktureinheit a2),
69,5 bis 97,5 Gew.-%, besonders bevorzugt 84,5 bis 96,5 Gew.-%, der Struktureinheit b) und
0,01 bis 5 Gew.-%, besonders bevorzugt 0,2 bis 3 Gew.-%, insbesondere bevorzugt 0,5 bis 2 Gew.-%, der Struktureinheit c).

Besonders bevorzugte Struktureinheiten gemäß Formel (1) leiten sich vom N-Vinylpyrrolidon ab.

Als Struktureinheit gemäß Formel (3) eignen sich bevorzugt Ammoniumsalze der 2-Acrylamido-2-methyl-propan-sulfonsäure, besonders bevorzugt das NH₄⁺-Salz.

Die vernetzenden Struktureinheiten c) leiten sich bevorzugt ab von Acryl- oder Methacryl-säureallylester, Trimethylolpropantriacrylat, Trimethylolpropanmethacrylat, Dipropylenglykoldiallylether, Polyglykoldiallylether, Triethylenglykoldivinylether, Hydrochinondiallylether, Tetraallyloxyethan oder anderen Allyl- oder Vinylethern multifunktioneller Alkohole, Tetraethylenglykoldiacrylat, Triallylamin, Trimethylolpropandiallylether, Methylenbisacrylamid und/oder Divinylbenzol. Besonders bevorzugt sind Acrylsäureallylester, Methacrylsäureallylester, Trimethylolpropantriacrylat und/oder Trimethylolpropanmethacrylat.
Insbesondere bevorzugt leiten sich die vernetzenden Strukturen ab von Monomeren der allgemeinen Formel (4) 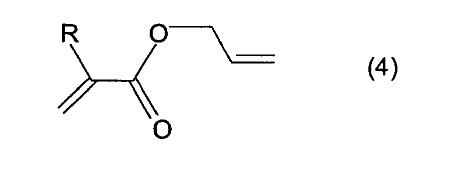 worin R Wasserstoff, Methyl oder Ethyl bedeutet.

Bevorzugt handelt es sich bei den Copolymeren um wasserlösliche oder wasserquellbare Copolymere.

Die Herstellung der Copolymere erfolgt bevorzugt durch radikalische Copolymerisation, bevorzugt durch Fällungspolymerisation, besonders bevorzugt in tert.-Butanol. Bevorzugt werden dabei die Monomeren entsprechend den Formeln (1), (2) und (3) in einem protischen Lösungsmittel gelöst oder dispergiert, anschließend werden zu dieser Lösung oder Dispersion ein oder mehrere Vernetzer c) gegeben und die Polymerisation in bekannter Weise durch Zugabe einer radikalbildenden Verbindung gestartet.
Die Polymerisationsreaktion erfolgt vorzugsweise in einem wasserlöslichen Alkohol oder einem Gemisch mehrerer Alkohole mit 1 bis 6 C-Atomen, vorzugsweise in tert.-Butanol. Der Wassergehalt des Alkohols oder Alkoholgemisches sollte 10 Gew.-% nicht überschreiten, da sonst im Verlauf der Polymerisation eine Klumpenbildung auftreten kann. Die Wahl der Art und der Menge des Lösungsmittels sollte so erfolgen, dass das Salz der Acrylamidoalkylsulfonsäure entsprechend Formel (3), insbesondere der 2-Acrylamido-2-methyl-propan-sulfonsäure, darin weitgehend löslich oder dispergierbar ist. Unter weitgehend löslich oder dispergierbar ist zu verstehen, dass sich auch nach Abstellen des Rührwerks kein festes Material aus der Lösung oder Dispersion absetzt. Das im Verlaufe der Reaktion entstehende Polymerisat soll hingegen in dem gewählten Lösungsmittel bzw. Lösungsmittelgemisch weitgehend unlöslich sein. Unter weitgehend unlöslich ist hierbei zu verstehen, dass im Verlauf der Polymerisation eine gut rührbare, breiige Polymerpaste entsteht, in der sich keine Klumpen oder Verklebungen bilden. Das durch Absaugen der Paste erhältliche Filtrat sollte einen Feststoffgehalt von maximal 5 Gew.-% aufweisen. Sind die Copolymere in stärkerem Ausmaß im gewählten Lösungsmittel oder Lösungsmittelgemisch löslich, kann es beim Trocknen der Polymerisatpaste zu Verklumpungen kommen.
Die Polymerisationsreaktion selbst wird in an sich bekannter Weise durch radikalbildende Verbindungen wie Azoinitiatoren (z.B. Azobisisobutyronitril), Peroxide (z.B. Dilaurylperoxid) oder Persulfate im Temperaturintervall von 20 bis 120°C, vorzugsweise zwischen 40 und 80°C, gestartet und über einen Zeitraum von 30 Minuten bis mehrere Stunden fortgeführt.
Das Eigenschaftsprofil der Copolymere lässt sich durch Variation des oben Mischungsverhältnisses der Monomere sowie der Vernetzer variieren. So kann z.B. durch den verstärkten Einbau von Ammoniumsalzen der Acrylamidosulfonsäuren die verdickende Wirkung der Polymerisate verbessert werden. Durch Einbau von mehr cyclischem N-Vinylcarbonsäureamid wird dagegen die Elektrolytverträglichkeit der Polymerisate und deren Löslichkeit in nicht-wässrigen Systemen verbessert.

Besonders bevorzugt werden als Acrylamidopropylsulfonsäure-Salze die NH₄⁺-Salze einpolymerisiert. Anstelle der Ammoniumsalze kann man auch die freien Acrylamidopropylsulfonsäuren einsetzen und vor der Zugabe der restlichen Monomere durch Einleiten von Ammoniak die Ammoniumsalze erzeugen.

Die erfindungsgemäßen Mittel enthalten bevorzugt 0,01 bis 10 Gew.-%, besonders bevorzugt 0,1 bis 5 Gew.-%, insbesondere bevorzugt 0,5 bis 3 Gew.-%, an Copolymeren.

Die Mittel können weiterhin Ölkörper, organische Lösemittel, kationische Polymere, Siliconverbindungen, Filmbildner, Überfettungsmittel, feuchtigkeitsspendende Mittel, Stabilisatoren, biogene Wirkstoffe, Glycerin, Konservierungsmittel, Perlglanzmittel, Farb- und Duftstoffe, Lösemittel, Hydrotrope, Trübungsmittel, Verdickungsmittel, Dispergiermittel, Eiweißderivate, wie z.B. Gelatine, Collagenhydrolysate, Polypeptide auf natürlicher und synthetischer Basis, Eigelb, Lecithin, Lanolin und Lanolinderivate, deodorierende Mittel, Stoffe mit keratolytischer und keratoplastischer Wirkung, Enzyme, Trägersubstanzen, Antioxidantien, UV-Lichtschutzfilter, Pigmente und Metalloxide, antimikrobiell wirkende Agentien und/oder saure Wirkstoffe enthalten.

Als Ölkörper eignen sich bevorzugt Silikonöle (flüchtig, nicht flüchtig, linear, verzweigt, ringförmig, gegebenenfalls organisch modifiziert), Phenylsilikone, Silikonharze und -gummis, Mineralöle (z.B. Paraffin- oder Vaselinöl), tierische Öle (z.B. Perhydrosqualen, Lanolin), pflanzliche Öle (flüssige Triglyceride, z.B. Sonnenblumen-, Mais-, Soja-, Reis-, Jojoba-, Babusscu-, Kürbis-, Traubenkern-, Sesam-, Walnuss-, Aprikosen-, Makadamia-, Avocado-, Süßmandel-, Wiesenschaumkraut-, Ricinusöl, Triglyceride der Capryl/Caprinsäuren, Olivenöl, Erdnussöl, Rapsöl und Kokosnussöl), synthetische Öle (z.B. Purcellinöl, Isoparaffine), lineare und/oder verzweigte Fettalkohole und Fettsäureester (bevorzugt Guerbetalkohole mit 6 bis 18, vorzugsweise 8 bis 10,
Kohlenstoffatomen), Ester von linearen (C₆-C₁₃)-Fettsäuren mit linearen (C₆-C₂₀)-Fettalkoholen, Ester von verzweigten (C₆-C₁₃)-Carbonsäuren mit linearen (C₆-C₂₀)-Fettalkoholen, Ester von linearen (C₆-C₁₈)-Fettsäuren mit verzweigten Alkoholen (bevorzugt 2-Ethylhexanol), Ester von linearen und/oder verzweigten Fettsäuren mit mehrwertigen Alkoholen (z.B. Dimerdiol oder Trimerdiol) und/oder Guerbetalkoholen, Triglyceride auf Basis von (C₆-C₁₀)-Fettsäuren, Ester (z.B. Dioctyladipate, Diisopropyl dimer dilinoleate, Propylenglycole/-dicaprilate), Wachse (z.B. Bienenwachs, Paraffinwachs oder Mikrowachse) gegebenenfalls in Kombination mit hydrophilen Wachsen (z.B. Cetylstearylalkohol), fluorierte und perfluorierte Öle; fluorierte Silikonöle und Gemische der vorgenannten Verbindungen.

Der Öl-Anteil der Mittel kann, bezogen auf die fertigen Mittel, bis 95 Gew.-%, bevorzugt 2 bis 95 Gew.-%, besonders bevorzugt 2 bis 50 Gew.-%, insbesondere bevorzugt 5 bis 20 Gew.-%, betragen.

Der Anteil an Ölkörper ist unter anderem abhängig davon, ob Mittel mit einer niedrigen Viskosität (z.B. Lotionen) oder Mittel mit einer hohen Viskosität (z.B. Cremes, Salben) erwünscht sind.

Als organische Lösemittel eignen sich bevorzugt ein- und mehrwertige Alkohole, gegebenenfalls ethoxylierte Polyethylenglykole, Propylenglykolester, Sorbit und dessen Derivate, Glykolether, Propylenglykolether und/oder Fettester.

Der Gehalt an organischen Lösemitteln kann bis 90 Gew.-%, bevorzugt 5 bis 70 Gew.-%, betragen.

Als kationische Polymere eignen sich die unter der INCI-Bezeichnung "Polyquaternium" bekannten Verbindungen, insbesondere Polyquaternium-31, Polyquaternium-16, Polyquaternium-24, Polyquaternium-7, Polyquaternium-22, Polyquaternium-39, Polyquaternium-28, Polyquaternium-2, Polyquaternium-10, Polyquaternium-11, sowie Polyquaternium 37&mineral oil&PPG trideceth (Salcare SC95), PVP-dimethylaminoethylmethacrylat-Copolymer, Guarhydroxypropyltriammoniumchloride, sowie Calciumalginat und Ammoniumalginat. Des weiteren eignen sich kationische Cellulosederivate; kationische Stärke; Copolymere von Diallylammoniumsalzen und Acrylamiden; quaternierte Vinylpyrrolidon/Vinylimidazol-Polymere; Kondensationsprodukte von Polyglykolen und Aminen; quaternierte Kollagenpolypeptide; quaternierte Weizenpolypeptide; Polyethylenimine; kationische Siliconpolymere (z.B. Amidomethicone); Copolymere aus Adipinsäure und Dimethylaminohydroxypropyldiethylentriamin; Polyaminopolyamide und kationische Chitinderivate (z.B. Chitosan).

Geeignete Siliconverbindungen sind beispielsweise Dimethylpolysiloxan, Methylphenylpolysiloxane, cyclische Silicone und amino-, fettsäure-, alkohol-, polyether-, epoyx-, fluor- und/oder alkylmodifizierte Siliconverbindungen, sowie Polyalkylsiloxane, Polyalkylarylsiloxane und Polyethersiloxan-Copolymere, die bei Raumtemperatur sowohl flüssig als auch harzförmig vorliegen können.

Geeignete Filmbildner sind, je nach Anwendungszweck wasserlösliche Polyurethane (z.B. C10-Polycarbamylpolyglycerylester), Polyvinylalkohol, Polyvinylpyrrolidon, Polyvinylpyrrolidoncopolymere (z.B. Vinylpyrrolidon/Vinylacetatcopolymere), wasserlösliche Acrylsäurepolymere/-copolymere bzw. deren Ester oder Salze, (z.B. Partialestercopolymere der Acryl/Methacrylsäure), Polyethylenglykolether von Fettalkoholen (z.B. Acrylat/Steareth-20-Methacrylat Copolymer), wasserlösliche Cellulose (z.B. Hydroxymethylcellulose, Hydroxyethylcellulose, Hydroxypropylcellulose), wasserlösliche Quaterniums, Polyquaterniums, Carboxyvinylpolymere, (z.B. Carbomere und deren Salze) und/oder Polysaccharide (z.B. Polydextrose, Glucan).

Als Überfettungsmittel eignen sich z.B. polyethoxylierte Lanolinderivate, Lecithinderivate, Polyolfettsäureester, Monoglyceride und Fettsäurealkanolamide, wobei letzteren gleichzeitig als Schaumstabilisatoren dienen.

Als feuchtigkeitsspendende Substanz stehen beispielsweise Isopropylpalmitat, Glycerin und/ oder Sorbitol zu Verfügung.

Als Stabilisatoren können Metallsalze von Fettsäuren, wie z.B. Magnesium-, Aluminium- und/oder Zinkstearat, eingesetzt werden.

Unter biogenen Wirkstoffen sind beispielsweise Pflanzenextrakte und Vitaminkomplexe zu verstehen.

Die erfindungsgemäßen Mittel können mit konventionellen Ceramiden, Pseudoceramiden, Fettsäure-N-alkylpolyhydroxyalkylamiden, Cholesterin, Cholesterinfettsäureestern, Fettsäuren, Triglyceriden, Cerebrosiden, Phospholipiden und ähnlichen Stoffen als Pflegezusatz abgemischt werden.

Als UV-Filter eignen sich z.B. 4-Aminobenzoesäure;
3-(4'-Trimethylammonium)benzyliden-boran-2-on-methylsulfat;
3,3,5-Trimethyl-cyclohexylsalicylat; 2-Hydroxy-4-methoxybenzophenon;
2-Phenylbenzimidazol-5-sulfonsäure und ihre Kalium-, Natrium- und
Triethanolaminsalze;
3,3'-(1,4-Phenylendimethin)-bis-(7,7-dimethyl-2-oxobicyclo[2.2.1]-heptan-1-methansulfonsäure und ihre Salze;
1-(4-tert.-Butylphenyl)-3-(4-methoxyphenyl)propan-1,3-dion,
3-(4'-Sulfo)-benzyliden-bornan-2-on und seine Salze;
2-Cyan-3,3-diphenyl-acrylsäure-(2-ethylhexylester);
Polymer von N-[2(und 4)-(2-oxoborn-3-ylidenmethyl)benzyl]-acrylamid;
4-Methoxy-zimtsäure-2-ethyl-hexylester;
ethoxyliertes Ethyl-4-amino-benzoat;
4-Methoxy-zimtsäure-isoamylester; 2,4,6-Tris-[p-(2-ethylhexyloxycarbonyl)anilino]-1,3,5-triazin;
2-(2H-benzotriazol-2-yl)-4-methyl-6-(2-methyl-3-(1,3,3,3-tetramethyl-1-(trimethylsilyloxy)-disiloxanyl)propyl)phenol;
4,4'-[(6-[4-((1,1-dimethylethyl)-amino-carbonyl)phenylamino]-1,3,5-triazin-2,4-yl)diimino]bis-(benzoesäure-2-ethylhexylester);
3-(4'-Methylbenzyliden)-D,L-Campher;
3-Benzyliden-Campher; Salicylsäure-2-ethylhexylester;
4-Dimethylaminobenzoesäure-2-ethylhexylester;
Hydroxy-4-methoxy-benzophenon-5-sulfonsäure (Sulisobenzonum) und das Natriumsalz; und/oder 4-Isopropylbenzylsalicylat.

Als Pigmente/Mikropigmente können z.B. mikrofeines Titandioxid, Glimmer-Titanoxid, Eisenoxide, Glimmer-Eisenoxid, Zinkoxid, Siliciumoxide, Ultramarinblau und Chromoxide eingesetzt werden.

Als Antioxidantien eignen sich beispielsweise Aminosäuren (z.B. Glycin, Histidin, Tyrosin, Tryptophan) und deren Derivate, Imidazole (z.B. Urocaninsäure und deren Derivate), Peptide (z.B. D,L-Carnosin, D-Carnosin, L-Carnosin und deren Derivate, wie z.B. Anserin), Carotinoide, Carotine(z.B. α-Carotin, β-Carotin, Lycopin und deren Derivate), Liponsäure und deren Derivate (z.B. Dihydroliponsäure, Aurothioglucose, Propylthiouracil), Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, gamma-Linoleyl-, Cholesteryl- und Glycerylester sowie deren Salze), Dilaurylthiodipropionat, Distearylthiodipropionat, Thiodipropionsäure und deren Derivate (z.B. Ester, Ether, Peptide, Lipide, Nukleotide, Nukleoside und Salze) und/oder Sulfoximinverbindungen (z.B. Buthioninsulfoximine), Homocysteinsulfoximin, Buthioninsulfone, Penta-, Hexa-, Heptathioninsulfoximin, Metall-Chelatoren (z.B. α-Hydroxyfettsäuren, Palmitinsäure, Phytinsäure, Lactoferrin), α-Hydroxysäuren (z.B. Zitronensäure, Milchsäure, Äpfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure, Folsäure und deren Derivate), Ubichinon und Ubichinol und deren Derivate, Vitamin C und dessen Derivate (z.B. Ascorbylpalmitate, Mg-Ascorbylphosphate, Ascorbylacetate), Tocopherol und dessen Derivate (z.B. VitaminE-acetat), Vitamin A und dessen Derivate (z.B. Vitamin-A-palmitat), Konyferylbenzoat des Benzoeharzes, Rutinsäure und deren Derivate, Ferulasäure und deren Derivate, Butylhydroxytoluol, Butylhydroxyanisol, Nordihydroguajakharzsäure, Nordihydroguajeretsäure, Trihydroxybutyrophenon, Harnsäure und deren Derivate, Mannose und deren Derivate, Zinkverbindungen (z.B. ZnO, ZnSO4), Selenverbindungen (z.B. Selenmethionin), Stilbene und deren Derivate (z.B. Stilbenoxid, Trans-Stilbenoxid).

Als Antioxidantien werden vorteilhafterweise öllösliche Verbindungen eingesetzt. Die Mitteln enthalten die Antioxidantien, bezogen auf die fertigen Mittel, bevorzugt in Mengen von 0,001 bis 30 Gew.-%, besonders bevorzugt 0,05 bis 20 Gew.-%, insbesondere bevorzugt 1 bis 10 Gew.-%.

Als Konservierungsmittel eignen sich z.B. Phenoxyethanol, Parabene, Pentandiol oder Sorbinsäure.

Als Farbstoffe können die für kosmetische Zwecke geeigneten und zugelassenen Substanzen verwendet werden.

Als fungizide Wirkstoffe eignen sich bevorzugt Ketoconazol, Oxiconazol, Bifonazole, Butoconazole, Cloconazole, Clotrimazole, Econazole, Enilconazole, Fenticonazole, Isoconazole, Miconazole, Sulconazole, Tioconazole Fluconazole, Itraconazole, Terconazole, Naftifine und Terbinafine, Zn-Pyrethion und Octopyrox.

Bevorzugte saure Wirkstoffe sind Glykolsäure, Milchsäure, Zitronensäure, Weinsäure, Mandelsäure, Salicylsäure, Ascorbinsäure, Brenztraubensäure, Oligooxa Mono- und Dicarbonsäuren, alpha-Hydroxysäuren, Fumarsäure, Retinoesäure, Sulfonsäuren, Benzoesäure, Kojisäure, Fruchtsäure, Äpfelsäure, Gluconsäure und/oder Derivate der vorgenannten Verbindungen.

Die Mittel werden bevorzugt auf einen pH-Wert im Bereich 2 bis 12, bevorzugt 3 bis 8, eingestellt.

In einer weiteren bevorzugten Ausführungsform der Erfindung besitzen die erfindungsgemäßen Mittel einen pH-Wert kleiner als 6,5.

Bevorzugt handelt es sich bei den Mitteln um Emulsionen vom Typ Öl-in-Wasser oder Wasser-in-Öl. Die Herstellung der Emulsionen kann in an sich bekannter Weise, d.h. beispielsweise durch Heiß-, Heiß/ Kalt- oder PIT- Emulgierung erfolgen.

Bevorzugt handelt es sich bei den Emulsionen um Tagescremes, Nachtcremes, Pflegecremes, Nährcremes, Reinigungsmilch, Hautmilch, Sonnenschutzlotion, Hautschutzcremes, Bodylotions, Salben und dergleichen.

Auf Grund ihrer guten Verträglichkeit eignen sich die erfindungsgemäßen Mittel besonders gut als Mittel zur Reinigung, Pflege oder medizinischen Behandlung der Haut, der Kopfhaut, der Haare, der Nägel oder der Schleimhäute.

Weiterhin eignen sich die erfindungsgemäßen Mittel gut als Vehikel für kosmetische und pharmazeutische Wirkstoffe für die Haut, wobei es sich bei den Wirkstoffen bevorzugt um Antioxidantien handelt, die die Haut vor oxidativer Beanspruchung schützen.

Die nachfolgenden Beispiele sollen die Erfindung näher erläutern, ohne sie jedoch darauf zu beschränken.

Herstellung der Copolymere:

### Beispiel 1:

In einem 1000 ml Kolben mit Ankerrührer, Rückflusskühler, Innenthermometer, Einleitungsmöglichkeit für N₂ und NH₃ werden 490,5 g tert. Butanol und 11,5 g Wasser vorgelegt. Anschließend wurden 80,75 g 2-Acrylamido-2-methyl-propansulfonsäure eingetragen und unter starkem Rühren dispergiert, wobei eine Trübung des Lösungsmittels erhalten bleibt. Über einen Zeitraum von 30 Minuten leitet man 6,64 g Ammoniak in den überstehenden Gasraum ein und rührt mindestens weitere 30 Minuten nach bis sich ein pH-Wert von 6-7 einstellt. Man gibt 4,10 g N-Vinylpyrrolidon und 0,8 g Methacrylsäureallylester hinzu und spült die Vorlage jeweils mit tert. Butanol (ca. 6 ml) nach, um Verluste bei der Zugabe zu minimieren. Das Reaktionsgemisch wird dann auf eine Temperatur von T = 60°C erwärmt, wobei die Reaktionsmischung durch gleichzeitiges Einleiten von N₂ inertisiert wird. Nach Erreichen der Temperatur von T = 60°C wird 1,0 g Dilaurylperoxid zugegeben. Die Reaktion springt unmittelbar nach Zugabe des Initiators an, was an einem Anstieg der Temperatur und am Ausflocken des Polymers zu erkennen ist.
Etwa 15 Minuten nach dem Einsetzen der Polymerisationsreaktion wird die Stickstoffzufuhr abgestellt. Ungefähr 30 Minuten nach Zugabe des Dilaurylperoxids erreicht die Temperatur ein Maximum (ca. 65 - 70°C). Weitere 30 Minuten nach Durchlaufen dieses Maximums wird zum Rückfluss erhitzt und unter diesen Bedingungen zwei Stunden nachgerührt. Der Inhalt des Reaktionsgefäßes nimmt im Verlauf der Reaktion eine breiartige Konsistenz an, ist aber noch gut rührbar. Anschließend wird auf Raumtemperatur abgekühlt und der Feststoff abgesaugt.
Die Paste wird bei 60 - 70°C über 24 Stunden im Vakuumtrockenschrank getrocknet. Man erhält 92,2 g eines feinen weißen Pulvers.

### Beispiel 2:

Reaktionsführung analog Beispiel 1, jedoch werden anstelle von Methacrylsäureallylester 1,65 g Trimethylolpropanmethacrylat eingesetzt.

### Beispiel 3:

Reaktionsführung analog Beispiel 1, jedoch werden als Monomere 35 g 2-Acrylamido-2-methyl-propan-sulfonsäure, 55 g N-Vinylpyrrolidon und 1,9 g Trimethylolpropantriacrylat eingesetzt.

### Beispiel 4:

Reaktionsführung analog Beispiel 1, jedoch werden als Monomere 77,5 g 2-Acrylamido-2-methyl-propan-sulfonsäure, 8,9 g N-Vinylpyrrolidon, 4,2 g N-Vinylformamid und 1,8 g Trimethylolpropantriacrylat eingesetzt.

### Anwendungen:

Säurestabilität der Copolymere:
Tabelle 1 belegt die drastisch höhere Säurestabilität der Copolymere im Vergleich zu einem ^{®}Carbopol-Typ auf Basis von Acrylsäure (Carbopol^{®} 934 der Fa. Goodrich).

**Tabelle 1: Säurestabilität der Copolymere**

| pH-Wert | Copolymer nach Beispiel 1 | ^{®}Carbopol 934 |
|---|---|---|
| 6-7 | 65 600 mPa·s | 76 600 mPa·s |
| ca. 3 | 52 100 mPa·s | 140 mPa·s |

Die Bestimmung der Säurestabilität erfolgte durch Viskositätsmessung bei 25°C mit dem Brookfield-Viskosimeter Typ RVT bei 20 Upm. Die Viskositätsbestimmung erfolgte an 1,0 %igen Gelen (Auflösung der Polymerpulver in destilliertem Wasser), deren pH-Wert gegebenenfalls durch Zugabe von NaOH bzw. Zitronensäure auf einem sauren (pH = ca. 3) und einen neutralen (pH = 6-7) pH-Wert eingestellt wurde.

### Lagerstabilität der erfindungsgemäßen Mittel:

Unter Verwendung der in Beispiel 1 bis 4 hergestellten Copolymere wurden entsprechend der unten angegebenen Formulierungsvorschrift die erfindungsgemäßen Mittel 1 bis 4 hergestellt. Für Vergleichszwecke wurde das Mittel 5 enthaltend ein ^{®}Carbopol 980 hergestellt.

Formulierungsvorschrift zur Herstellung der Mittel 1 bis 5 (alle Angaben in Gew.-%):

| | | |
|---|---|---|
| A | Almond Oil | 7,00 % |
| | ^{®}Dow Corning 345 Cyclomethicon | 5,00 % |
| B | Copolymere gemäß Bsp. 1 bis 4 bzw. ^{®}Carbopol 980 | 1,00 % |
| C | Wasser | ad 100 % |
| | Glycerin | 8,00 % |
| | Preservative (Nipa) | q.s. |
| D | Fragrance | 0,30 % |

### Herstellung

I A und B mischen
II. Die Komponenten von C mischen.
III II zu I hinzugeben
IV D zu I hinzurühren
V Emulsion homogenisieren, pH 4,5

Folgende Stabilitätstests werden durchgeführt :
a) Lagerung bei 40°C, 45°C und 50°C über einen Zeitraum von 90 Tagen.
b) Zentrifugieren bei 20°C für 30 Minuten bei 5000 U/min.

Die Ergebnisse der Stabilitätstest für die Mittel 1 bis 5 sind in Tabelle 2 zusammengefasst.

**Tabelle 2: Stabilität der Mittel 1 bis 5**

| | Lagerung 40°C | Lagerung 45°C | Lagerung 50°C | Zentrifugentest |
|---|---|---|---|---|
| Mittel 1 (Copolymer Bsp. 1) | stabil | stabil | stabil | stabil |
| Mittel 2 (Copolymer Bsp. 2) | stabil | stabil | stabil | stabil |
| Mittel 3 (Copolymer Bsp. 3) | stabil | stabil | instabil | stabil |
| Mittel 4 (Copolymer Bsp.4) | stabil | stabil | stabil | stabil |
| Mittel 5, Vergleich (^{®}Carbopol 980) | instabil | instabil | instabil | stabil |

Die erfindungsgemäßen Mittel 1 bis 4 zeigen eine gute Emulsionsstabilität bei Lagerung bei 40°C, 45°C und 50°C über einen Zeitraum von 90 Tagen und beim Zentrifugieren (20°C, 30 Minuten, 5000 U/min). Weiterhin sind die erfindungsgemäßen Mittel leicht und frisch und besitzen feuchtigkeitsspendende Eigenschaften. Das Hautgefühl ist angenehm glatt und samtig.
Das Creme-Gel zieht schnell in die Haut ein und ist weder ölig noch klebrig.

Die Vergleichsformulierung 5 mit ^{®}Carbopol 980 ist nicht lagerstabil. Bei Zugabe von 3 Gew.-% Emulgator (^{®}Hostaphat KL 340 D, Mono-, di- and tri-(alkyltetraglycol ether)-o-phosphoric acid esters, Clariant) wird eine O/W Emulsion erhalten, die im Lagertest stabil ist.

### Anwendungsbeispiele 5 bis 9:

### Beispiel 5: Tensidfreie O/W - Hautmilch mit keratolytischer Wirkung

| | | |
|---|---|---|
| A | Copolymer aus Beispiel 1 | 2,00 % |
| | Mineralöl | 4,00 % |
| | Mandelöl | 4,00 % |
| | ^{®}Cetiol SN (Henkel) Cetearylisononanoat | 2,00 % |
| C | Wasser | ad 100 % |
| | Zitronensäure | 0,30 % |
| | Äpfelsäure | 0,40 % |
| | Glykolsäure | 0,70 % |
| | Milchsäure | 0,70 % |
| D | Duftstoffe | 0,30 % |

| Herstellung | |
|---|---|
| I | Komponenten von C mischen und zu A geben |
| II | D zu I hinzurühren |
| III | Emulsion homogenisieren, pH 3,5 |

### Beispiel 6: Tensidfreie feuchtigkeitsspendende Lotion

| | | |
|---|---|---|
| A | Almondöl | 7,00 % |
| | Cyclomethicone | 5,00 % |
| B | Copolymer aus Beispiel 2 | 1,50 % |
| C | Glycerin | 7,00 % |
| | Water | ad 100 % |
| | Konservierungsmittel | q.s. |
| D | Duftstoff | 0,30 % |

| Herstellung | |
|---|---|
| I | A und B mischen. |
| II | Lösung von C in I einrühren. |
| III | D zu II geben. |
| IV | homogenisieren |
| V | pH 5,5 |

### Beispiel 7: Tensidfreie erfrischende Lotion

| | | |
|---|---|---|
| A | Almondöl | 5,00 % |
| | Cyclomethicon | 5,00 % |
| B | Copolymer aus Beispiel 4 | 1,50 % |
| C | Glycerin | 3,00 % |
| | Ethanol | 20,00 % |
| | Wasser | ad 100 % |
| | Konservierungsmittel | q.s. |
| D | Duftstoff | 0,30 % |

| Herstellung | |
|---|---|
| I | A und B mischen. |
| II | Lösung von C in I einrühren. |
| III | D zu II zufügen. |
| IV | homogenisieren |

### Beispiel 8: Tensidfreie Lotion mit erfrischender, belebender Wirkung

| | | |
|---|---|---|
| A | Jojoba oil | 3,00 % |
| | Almondöl | 3,00 % |
| | Cetiol V | 3,00 % |
| | Decyloleat | |
| B | Copolymer aus Beispiel 1 | 1,50 % |
| C | Glycerin | 3,00 % |
| | Menthol | 0,70% |
| | Campher | 0,30% |
| | Ethanol | 5,00% |
| | Wasser | ad 100 % |
| | Konservierungsmittel | q.s. |
| D | Duftstoff | 0,30 % |

| Herstellung | |
|---|---|
| I | A und B mischen. |
| II | Lösung von C in I einrühren. |
| III | D zu II geben. |
| IV | homogenisieren |

### Beispiel 9: Tensidfreie Sonnenschutzlotion

| | | |
|---|---|---|
| A | Vaselin | 5,00% |
| | Paraffinöl | 10,00% |
| | Copolymer aus Beispiel 1 | 0,75 % |
| | Tocopherylacetat | 1,00 % |
| | Octylmethoxycinnamat | 2,00 % |
| | Parasol 1789 | 0,20 % |
| B | Ethanol | 10,00 % |
| C | Butylenglycol | 5,00 % |
| | Wasser | ad 100 % |

| Herstellung | |
|---|---|
| I | A und C getrennt auf 75°C erwärmen, danach A und C vereinigen und unter Rühren auf 65°C abkühlen, homogenisieren und weiter auf 35°C abkühlen |
| II | B in I einrühren, homogenisieren und auf Raumtemperatur abkühlen |

## Patentansprüche

1. Tensidfreie, kosmetische, dermatologische und pharmazeutische Mittel, **dadurch gekennzeichnet, dass** sie mindestens ein Copolymer enthalten, bestehend im wesentlichen aus
a1) 1 bis 50 Gew.-% der wiederkehrenden Struktureinheit der Formel (1) 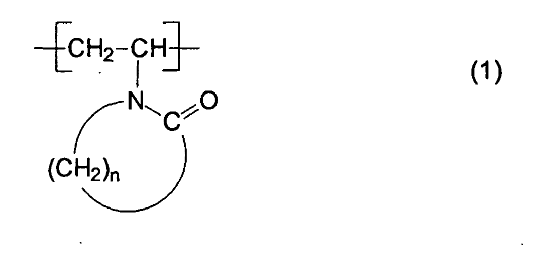 wobei n eine ganze Zahl von 2 bis 9 bedeutet,
oder
a2) 1 bis 50 Gew.-% einer Mischung der wiederkehrenden Struktureinheit der Formel (1) und der wiederkehrenden Struktureinheit der Formel (2) 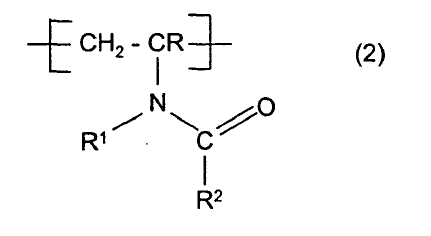 wobei R, R¹ und R² gleich oder verschieden sein können und Wasserstoff oder eine lineare oder verzweigte Alkyl- oder Alkenylgruppe mit jeweils 1 bis 30 C-Atomen bedeuten
und
b) 49,99 bis 98,99 Gew.-% der wiederkehrenden Struktureinheit der Formel (3) 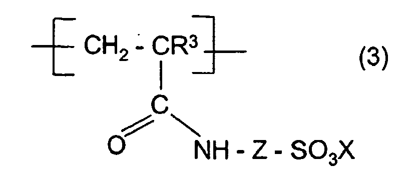 worin R³ Wasserstoff, Methyl oder Ethyl, Z (C₁-C₈)-Alkylen, n eine ganze Zahl von 2 bis 9 und X ein Ammonium-, Alkali- oder Erdalkali-lon bedeuten und
c) 0,01 bis 8 Gew.-% vernetzenden Strukturen, die aus Monomeren mit mindestens zwei olefinischen Doppelbindungen hervorgegangen sind.

2. Mittel nach Anspruch 1, **dadurch gekennzeichnet, dass** die Copolymere im wesentlichen 2 bis 30 Gew.-% der Struktureinheiten a1) oder a2), 69,5 bis 97,5 Gew.-% der Struktureinheit b) und 0,01 bis 5 Gew.-% der Struktureinheit c) enthalten.

3. Mittel nach Anspruch 2, **dadurch gekennzeichnet, dass** die Copolymere im wesentlichen 3 bis 15 Gew.-% der Struktureinheiten a1) oder a2), 84,5 bis 96,5 Gew.-%, der Struktureinheit b) und 0,2 bis 3 Gew.-% der Struktureinheit c) enthalten.

4. Mittel nach mindestens einem der Ansprüche 1 bis 3, **dadurch gekennzeichnet, dass** die Copolymere die Struktureinheit a2) enthalten.

5. Mittel nach mindestens einem der Ansprüche 1 bis 4, **dadurch gekennzeichnet, dass** sich die Struktureinheit gemäß Formel (1) vom N-Vinylpyrrolidon ableitet.

6. Mittel nach mindestens einem der Ansprüche 1 bis 5, **dadurch gekennzeichnet, dass** es sich bei X in der Struktureinheit b) um NH₄⁺ handelt.

7. Mittel nach mindestens einem der Ansprüche 1 bis 6, **dadurch gekennzeichnet, dass** sich die Struktureinheit c) von Acrylsäureallylester, Methacrylsäureallylester, Trimethylolpropantriacrylat und/oder Trimethylolpropanmethacrylat ableitet.

8. Mittel nach mindestens einem der Ansprüche 1 bis 7, **dadurch gekennzeichnet, dass** sie, bezogen auf die fertigen Mittel, 0,01 bis 10 Gew.-% der Copolymere enthalten.

9. Mittel nach mindestens einem der Ansprüche 1 bis 8, **dadurch gekennzeichnet, dass** sie Ölkörper, organische Lösemittel, kationische Polymere, Siliconverbindungen, Filmbildner, Überfettungsmittel, feuchtigkeitsspendende Mittel, Stabilisatoren, biogene Wirkstoffe, Glycerin, Konservierungsmittel, Perlglanzmittel, Farb- und Duftstoffe, Lösemittel, Hydrotrope, Trübungsmittel, Verdickungsmittel, Dispergiermittel, Eiweißderivate, Collagenhydrolysate, Polypeptide, Eigelb, Lecithin, Lanolin und Lanolinderivate, deodorierende Mittel, Stoffe mit keratolytischer und keratoplastischer Wirkung, Enzyme, Trägersubstanzen, Antioxidantien, UV-Lichtschutzfilter, Pigmente und Metalloxide, antimikrobiell wirkende Agentien und/oder saure Wirkstoffe enthalten.

10. Mittel nach mindestens einem der Ansprüche 1 bis 9, **dadurch gekennzeichnet, dass** sie 2 bis 95 Gew.-% Ölkörper enthalten.

11. Mittel nach mindestens einem der Ansprüche 1 bis 10, **dadurch gekennzeichnet, dass** sie 5 bis 70 Gew.-% organische Lösemittel enthalten.

12. Mittel nach mindestens einem der Ansprüche 1 bis 11, **dadurch gekennzeichnet, dass** sie einen pH-Wert von 2 bis 12 aufweisen.

13. Mittel nach Anspruch 12, **dadurch gekennzeichnet, dass** sie einen pH-Wert kleiner als 6.5 besitzen.

14. Mittel nach mindestens einem der Ansprüche 1 bis 13, **dadurch gekennzeichnet, dass** es sich dabei um eine Emulsion handelt.

15. Emulsion nach Anspruch 14, **dadurch gekennzeichnet, dass** es sich dabei um eine Nachtcreme, Pflegecreme, Nährcreme, Reinigungsmilch, Hautmilch, Sonnenschutzlotion, Hautschutzcreme, Bodylotions oder Salbe handelt.

16. Mittel nach mindestens einem der Ansprüche 1 bis 15, **dadurch gekennzeichnet, dass** es sich dabei um ein Mittel zur Reinigung, Pflege oder medizinischen Behandlung der Haut, der Kopfhaut, der Haare, der Nägel oder der Schleimhäute handelt.

## Claims

1. A surfactant-free cosmetic, dermatological or pharmaceutical composition which comprises at least one copolymer consisting essentially of
a1) 1 to 50% by weight of the repeat structural unit of the formula (1) 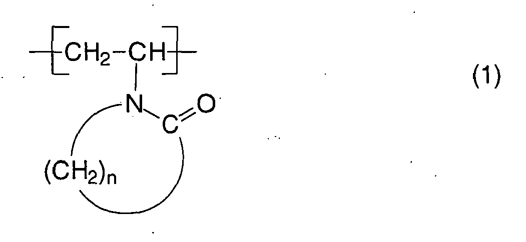 where n is an integer from 2 to 9,
or
a2) 1 to 50% by weight of a mixture of the repeat structural unit of the formula (1) and the repeat structural unit of the formula (2) 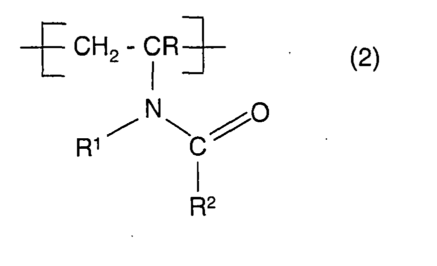 where R, R¹ and R² may be identical or different and are hydrogen or a linear or branched alkyl or alkenyl group having in each case 1 to 30 carbon atoms
and
b) 49.99 to 98.99% by weight of the repeat structural unit of the formula (3) 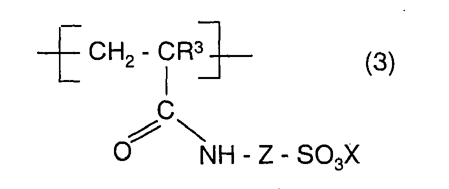 in which R³ is hydrogen, methyl or ethyl, Z is (C₁-C₈)-alkylene, n is an integer from 2 to 9 and X is an ammonium, alkali metal or alkaline earth metal ion
and
c) 0.01 to 8% by weight of crosslinking structures which originate from monomers having at least two olefinic double bonds.

2. The composition as claimed in claim 1, wherein the copolymers essentially comprise 2 to 30% by weight of the structural units a1) or a2), 69.5 to 97.5% by weight of the structural unit b) and 0.01 to 5% by weight of the structural unit c).

3. The composition as claimed in claim 2, wherein the copolymers essentially comprise 3 to 15% by weight of the structural units a1) or a2), 84.5 to 96.5% by weight of the structural unit b) and 0.2 to 3% by weight of the structural unit c).

4. The composition as claimed in at least one of claims 1 to 3, wherein the copolymers comprise the structural unit a2).

5. The composition as claimed in at least one of claims 1 to 4, wherein the structural unit according to formula (1) is derived from N-vinylpyrrolidone.

6. The composition as claimed in at least one of claims 1 to 5, wherein X in the structural unit b) is NH₄⁺.

7. The composition as claimed in at least one of claims 1 to 6, wherein the structural unit c) is derived from allyl acrylate, allyl methacrylate, trimethylolpropane triacrylate and/or trimethylolpropane methacrylate.

8. The composition as claimed in at least one of claims 1 to 7, which comprises, based on the finished composition, 0.01 to 10% by weight of the copolymers.

9. The composition as claimed in at least one of claims 1 to 8, which comprises oily substances, organic solvents, cationic polymers, silicone compounds, film formers, superfatting agents, moisturizing agents, stabilizers, biogenic active ingredients, glycerol, preservatives, pearlizing agents, dyes and fragrances, solvents, hydrotropic agents, opacifiers, thickeners, dispersants, protein derivatives, collagen hydrolysates, polypeptides, egg yolk, lecithin, lanolin and lanolin derivatives, deodorizing agents, substances with a keratolytic and keratoplastic action, enzymes, carrier substances, antioxidants, UV light protection filters, pigments and metal oxides, agents with an antimicrobial action and/or acidic active ingredients.

10. The composition as claimed in at least one of claims 1 to 9, which comprises 2 to 95% by weight of oily substances.

11. The composition as claimed in at least one of claims 1 to 10, which comprises 5 to 70% by weight of organic solvents.

12. The composition as claimed in at least one of claims 1 to 11, which has a pH of from 2 to 12.

13. The composition as claimed in claim 12, which has a pH of less than 6.5.

14. The composition as claimed in at least one of claims 1 to 13 which is in the form of an emulsion.

15. The emulsion as claimed in claim 14, which is in the form of a night cream, care cream, nutrient cream, cleansing milk, skin milk, sunscreen lotion, skin protection cream, body lotion or ointment.

16. The composition as claimed in at least one of claims 1 to 15, which is in the form of a composition for the cleansing, care or medicinal treatment of the skin, scalp, hair, nails or mucous membranes.

## Revendications

1. Agents cosmétiques, dermatologiques et pharmaceutiques exempts de tensio-actifs, **caractérisés en ce qu'**ils contiennent au moins un copolymère, essentiellement composé de
a1) 1% à 50% en poids du motif structurel récurrent de formule (1) 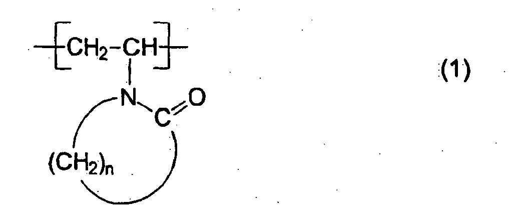 dans laquelle n représente un nombre entier de 2 à 9,
ou
a2) 1% à 50% en poids d'un mélange du motif structurel récurrent de formule (1) et du motif structurel récurrent de formule (2) 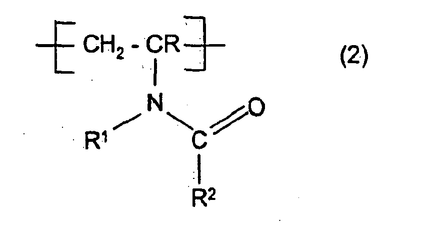 dans laquelle R, R¹ et R² peuvent être identiques ou différents et représentent un atome d'hydrogène ou un groupe alkyle ou alkylène linéaire ou ramifié contenant chacun 1 à 30 atomes de carbone
et
b) 49,99% à 98,99% en poids du motif structurel récurrent de formule (3) 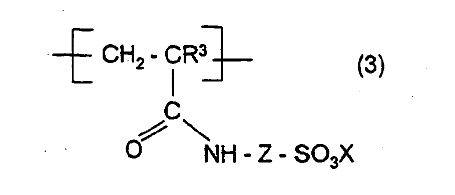 dans laquelle R³ représente un atome d'hydrogène, un groupe méthyle ou éthyle, Z représente un groupe alkylène(en C₁ à C₈), n représente un nombre entier de 2 à 9 et X représente un ion ammonium, alcalin ou alcalino-terreux
et
c) 0,01% à 8% en poids de structures réticulées qui proviennent de monomères contenant au moins deux doubles liaisons oléfiniques.

2. Agents selon la revendication 1, **caractérisés en ce que** les copolymères contiennent essentiellement 2% à 30% en poids de motif structurel a1) ou a2), 69,5% à 97,5% en poids de motif structurel b) et 0,01% à 5% en poids de motif structurel c).

3. Agents selon la revendication 2, **caractérisés en ce que** les copolymères contiennent essentiellement 3% à 15% en poids de motif structurel a1) ou a2), 84,5% à 96,5% en poids de motif structurel b) et 0,2% à 3% en poids de motif structurel c).

4. Agents selon au moins l'une quelconque des revendications 1 à 3, **caractérisés en ce que** les copolymères contiennent le motif structurel a2).

5. Agents selon au moins l'une quelconque des revendications 1 à 4, **caractérisés en ce que** le motif structurel de formule (1) est dérivé de la N-vinylpyrrolidone.

6. Agents selon au moins l'une quelconque des revendications 1 à 5, **caractérisés en ce que** X, dans le motif structurel b), représente NH₄⁺.

7. Agents selon au moins l'une quelconque des revendications 1 à 6, **caractérisés en ce que** le motif structurel c) est dérivé de l'ester allylique d'acide acrylique, de l'ester allylique d'acide méthacrylique, de triacrylate de triméthylolpropane et/ou de méthacrylate de triméthylolpropane.

8. Agents selon au moins l'une quelconque des revendications 1 à 7, **caractérisés en ce qu'**ils contiennent, par rapport aux agents finis, 0,01% à 10% en poids de copolymères.

9. Agents selon au moins l'une quelconque des revendications 1 à 8, **caractérisés en ce que** qu'ils contiennent des corps gras, des solvants organiques, des polymères cationiques, des composés de silicone, des agents filmogènes, des agents surgraissants, des agents hydratants, des stabilisants, des substances biogènes, de la glycérine, des agents conservateurs, des agents nacrants, des substances colorantes et odorantes, des solvants, des agents hydrotropes, des opacifiants, des épaississants, des dispersants, des dérivés protéiques, des hydrolysats de collagène, des polypeptides, du jaune d'oeuf, de la lécithine, de la lanoline et des dérivés de lanoline, des agents désodorisants, des substances ayant une action kératolytique et kératoplastique, des enzymes, des véhicules, des antioxydants, des filtres de protection anti-UV, des pigments et des oxydes métalliques, des agents ayant une action antimicrobienne et/ou des substances acides.

10. Agents selon au moins l'une quelconque des revendications 1 à 9, **caractérisés en ce qu'**ils contiennent 2% à 95% en poids de corps gras.

11. Agents selon au moins l'une quelconque des revendications 1 à 10, **caractérisés en ce qu'**ils contiennent 5% à 70% en poids de solvants organiques.

12. Agents selon au moins l'une quelconque des revendications 1 à 11, **caractérisés en ce qu'**ils présentent un pH de 2 à 12.

13. Agents selon la revendication 12, **caractérisés en ce qu'**ils possèdent un pH inférieur à 6,5.

14. Agents selon au moins l'une quelconque des revendications 1 à 13, **caractérisés en ce qu'**il s'agit d'une. émulsion.

15. Émulsion selon la revendication 14, **caractérisée en ce qu'**il s'agit d'une crème de nuit, d'une crème de soin, d'une crème nourrissante, d'un lait nettoyant, d'un lait pour le corps, d'une lotion dé protection solaire, d'une crème dermoprotectrice, d'une lotion pour le corps ou d'une pommade.

16. Agents selon au moins l'une quelconque des revendications 1 à 15, **caractérisés en ce qu'**il s'agit d'un agent pour le nettoyage, le soin ou le traitement médical de la peau, du cuir chevelu, des cheveux, des ongles ou des muqueuses.
